# EUROPEAN PATENT APPLICATION

(11) **EP 1 942 115 A1**
(43) Date of publication of application: **09.07.2008**
(21) Application number: 06822349.4
(22) Date of filing: 26.10.2006
(51) Int. Cl.: C07K 14/605, A61K 38/26, A61P 3/04, A61P 3/10

(54) **AGGLUTINABLE GLP-1 ANALOGUE AND SUSTAINED-RELEASE PHARMACEUTICAL COMPOSITION**

(30) Priority: 26.10.2005 JP 2005310658
(71) Applicant: CHUGAI SEIYAKU KABUSHIKI KAISHA, Tokyo, 115-8543 (JP)
(72) Inventor: KONISHI, Hiroko, Gotenba-shi, Shizuoka, 412-8513 (JP); IGAWA, Tomoyuki, Gotenba-shi, Shizuoka, 412-8513 (JP); SHIMOBOJI, Tsuyoshi, Gotenba-shi, Shizuoka, 412-8513 (JP); HIRAKURA, Tai, Gotenba-shi, Shizuoka, 412-8513 (JP)
(74) Representative: Harding, Charles Thomas
(86) International application number: PCT/JP2006/321373
(87) International publication number: WO 2007/049695

(57) **Abstract**

The present invention provides a GLP-1 analogue having a high association-aggregability or a pharmaceutically acceptable salt thereof, and a pharmaceutical composition to be used for preventing or treating diabetes, hyperglycemia, a diabetic complication caused by diabetes or hyperglycemia, or obesity, using the same.

## Description

### Technical Field

The present invention relates to an aggregable GLP-1 analogue, which is useful as a pharmaceutical preparation for preventing or treating diabetes, hyperglycemia, diabetic complications caused by diabetes or hyperglycemia and obesity, and a pharmaceutical composition containing the aggregable GLP-1 analogue.

### Background Art

Glucagon-like peptide-1 (GLP-1) is a peptide produced from proglucagon of 160 amino acid residues by a processing enzyme in small intestinal L cells and secreted in response to food intake. Other than a peptide consisting of a sequence of amino acid Nos. 72 to 108 of proglucagon (corresponding to GLP-1(1-37)), GLP-1(7-37) in which the amino acid residues at the 1- to 6-positions in the N-terminal region of the peptide are removed and GLP-1(7-36)NH₂ in which the C-terminus at the 36-position is amidated are known to act on pancreatic β-cells to induce insulin secretion thereby to decrease the serum glucose level (Non-patent document 1). It is known that because this action is not observed when the blood glucose level is low, the risk of low blood glucose level is low, and further, GLP-1 has an action of proliferating β-cells that produce insulin and inducing differentiation from progenitor cells, an action of inhibiting glucagon secretion, an action of delaying gastric emptying, an action of inhibiting food intake and the like (Non-patent document 1), therefore, expectation of applying GLP-1 to a therapeutic agent for diabetes is high. However, because of degradation by dipeptidyl peptidase-IV (DPPIV) and excretion from the kidney, the plasma half-life of GLP-1 is as short as several minutes only, therefore, frequent administration is necessary for using it as a pharmaceutical for diabetes. Various GLP-1 relatives and derivatives (analogues) (such as [Gly⁸]-GLP-1(7-37)) in which their biological activities are maintained and DPPIV resistance is imparted have been reported (Patent documents 1 to 8), however, the renal excretion cannot be avoided, therefore, the prolongation of retention in blood is not large.

Further, there is also a report of a sustained-release preparation of a GLP-1 analogue (Patent document 9), however, the release period is not longer than one day, which is short.
Patent document 1: WO 91/11457
Patent document 2: JP-A-11-310597
Patent document 3: WO 99/43705
Patent document 4: WO 00/69911
Patent document 5: WO 95/31214
Patent document 6: WO 00/07617
Patent document 7: WO 03/103572
Patent document 8: WO 97/29180
Patent document 9: JP-A-7-2695
Non-patent document 1: Int. J. Peptide Protein Research, Vol. 40, pp. 333-343, 1992

### Disclosure of the invention

### Problems that the Invention is to Solve

It is intended to provide an aggregable GLP-1 analogue for sustained-release use, which has a high activity and an unprecedented property of association-aggregability for achieving long-term sustained release, and is useful as a pharmaceutical preparation to be used for preventing or treating a disease selected from diabetes, hyperglycemia, diabetic complications caused by diabetes or hyperglycemia and obesity, and a preventive agent or a therapeutic agent containing the aggregable GLP-1 analogue for diabetes, hyperglycemia, a diabetic complication caused by diabetes or hyperglycemia or obesity.

### Means for Solving the Problems

In order to achieve the above object, the present inventors made intensive studies of searching a variant in which the association-aggregability has been improved without decreasing the biological activity, and as a result, they found that a GLP-1 analogue in which alanine at the 30-position of human GLP-1 or an analogue thereof has been substituted with arginine has an unprecedented property of association-aggregability, and further found that a substance obtained by aggregation thereof shows long-term release behavior, and thus, completed the present invention.

That is, according to one aspect of the present invention, a GLP-1 analogue in which alanine at the 30-position (Ala³⁰) of glucagon-like peptide-1 (GLP-1) or a GLP-1 analogue is Lys (Lys³⁰) or arginine (Arg³⁰) or a pharmaceutically acceptable salt thereof is provided.

According to another aspect of the present invention, the GLP-1 analogue or a pharmaceutically acceptable salt thereof according to claim 1, in which the alanine at the 30-position (Ala³⁰) of glucagon-like peptide-1 (GLP-1) or a GLP-1 analogue has been substituted with arginine (Arg³⁰) is provided.

According to still another aspect of the present invention, a GLP-1 analogue ([Arg³⁰]-GLP-1 analogue), in which alanine at the 30-position (Ala³⁰) of glucagon-like peptide-1 (GLP-1) or a GLP-1 analogue has been substituted with arginine (Arg³⁰), and which is used for preventing or treating a disease selected from diabetes, hyperglycemia, diabetic complications caused by diabetes or hyperglycemia and obesity is provided. The [Arg³⁰]-GLP-1 analogue of the present invention is an analogue having the biological activity of natural human GLP-1(7-37) and also aggregability, preferably an analogue having an in vitro biological activity equal to or higher than that of natural human GLP-1(7-37), more preferably an analogue in which the ratio of residual monomer is 20% or less in an evaluation test for aggregability.

Here, the GLP-1 and GLP-1 analogue to be substituted are not particularly limited, however, GLP-1 having an amino acid sequence derived from a mammal, particularly a human (hGLP-1) or an analogue thereof is preferred. Further, they may be a protein or a peptide consisting of an arbitrary fragment of proglucagon and having an incretin activity, or a protein or a peptide having an amino acid sequence in which one or several amino acids have been deleted, substituted or added in the sequence of the fragment. The GLP-1 analogue is not particularly limited, however, examples thereof include peptides having an amino acid sequence in which one or several (for example, 1 to 4) amino acids have been deleted, substituted or added in the sequence of GLP-1(1-37), GLP-1(7-37), GLP-1(7-36), GLP-1(1-37)NH₂, GLP-1(7-37)NH₂, GLP-1(7-36)NH₂ or the like.

The GLP-1 analogue is preferably an analogue in which a natural or non-natural amino acid mutation has been introduced for imparting resistance to a metabolic enzyme such as DPPIV in the body, other stability or the like. With regard to the position of the amino acid sequence at which such a mutation is introduced, a GLP-1 analogue in which an amino acid at the 7-, 8-, 9-, 10-, 15-, 16-, 18-, 21-, 22-, 23-, 24-, 26-, 31-, 34-, 35-, 36-position or the like has been substituted is known (such as WO 98/19698, WO 91/11457, and WO 00/34331), and also in the GLP-1 analogue of the present invention, one or more amino acids in such an amino acid sequence may be substituted with another natural or non-natural amino acid. As the analogue in which the metabolism is inhibited, an analogue in which Ala at the 8-position has been substituted with a natural or non-natural amino acid such as Gly, Ser, Val, Leu, Ile, Thr, Lys, Cys, Sar, D-alanine, β-alanine, Aib(α- aminoisobutyric acid), N-methyl-alanine, N-methyl-D- alanine, N-ethyl-D-alanine, N-methyl-glycine, 2-methyl- azetidine-2-carboxylic acid, α-methyl-(L)-proline, 2-methylpiperidine-2-carboxylic acid, isovaline, 1- aminocyclopropane carboxylic acid, 1-aminocyclobutane carboxylic acid, 1-aminocyclopentane carboxylic acid, 1-aminocyclohexane carboxylic acid, 1-aminocycloheptane carboxylic acid or 1-aminocyclooctane carboxylic acid is preferred, and particularly, an analogue in which Ala at the 8-position has been substituted with Gly, Ser, Val, Leu, Ile, Thr or Aib(α-aminoisobutyric acid) is more preferred.

Examples of the GLP-1 and the GLP-1 analogue include GLP-1(7-37), GLP-1(7-36)NH₂, [Gly⁸] -GLP-1(7-37), [Gly⁸] -GLP-1(7-36)NH₂, [Ser⁸]-GLP-1(7-37), [Ser⁸]-GLP-1 (7-36)NH₂, [Val⁸]-GLP-1 (7-37), [Val⁸]-GLP-1 (7-36)NH₂, [Leu⁸]-GLP-1(7-37), [Leu⁸]-GLP-1(7-36)NH₂, [Ile⁸]-GLP-1(7-37), [Ile⁸]-GLP-1(7-36)NH₂, [Thr⁸]-GLP-1(7-37), [Thr⁸] -GLP-1(7-36)NH₂, [Aib⁸]-GLP-1(7-36)NH₂, [Aib^{8,35}]-GLP-1(7-36)NH₂ and the like. Preferred examples thereof include [Gly⁸]-GLP-1(7-37), [Gly⁸]-GLP-1(7-36)NH₂, [Aib⁸]-GLP-1(7-36)NH₂, and [Aib^{8,35}]-GLP-1(7-36)NH₂.

In an embodiment of the present invention, the amino acid sequence of the GLP-1 analogue according to the present invention is [Gly⁸]-[Arg³⁰]-GLP-1(7-37) represented by the following sequence: His-Gly-Glu-Gly-Thr-Phe-Thr-Ser-Asp-Val-Ser-Ser-Tyr-Leu-Glu-Gly-Gln-Ala-Ala-Lys-Glu-Phe-Ile-Arg-Trp-Leu-Val-Lys-Gly-Arg-Gly (SEQ ID NO: 1).

According to another aspect of the present invention, a pharmaceutical composition containing the above-mentioned GLP-1 analogue is provided. According to one aspect of the present invention, the above-mentioned GLP-1 analogue may be associated and aggregated. This association and aggregation can be achieved by physical stress such as shaking or stirring, or a general method used for crystallization, amorphization or the like.

Further, the association and aggregation can also be achieved by allowing the GLP-1 analogue to coexist with a metal ion. Therefore, the GLP-1 analogue of the present invention may form an association aggregate or a metal complex containing a metal ion. The metal ion is selected from, for example, zinc, copper, iron, manganese, calcium, nickel, aluminum, sodium and potassium. The GLP-1 analogue of the present invention can form an aggregate with a remarkably small amount of metal ion compared with a wild type as shown in Examples mentioned below, and has a preferred property as a sustained-release pharmaceutical composition.

In an embodiment of the present invention, the above-mentioned pharmaceutical composition can be used for preventing or treating a disease selected from diabetes (insulin-dependent diabetes mellitus (type 1 diabetes) or noninsulin-dependent diabetes mellitus (type 2 diabetes)), hyperglycemia, diabetic complications caused by diabetes or hyperglycemia and obesity. Further, in another embodiment of the present invention, the pharmaceutical composition can be used as a sustained-release pharmaceutical composition.

According to a still another aspect of the present invention, a method for preventing or treating a disease selected from diabetes such as insulin-dependent diabetes mellitus (type 1 diabetes) or noninsulin-dependent diabetes mellitus (type 2 diabetes), hyperglycemia, diabetic complications caused by diabetes or hyperglycemia and obesity, comprising administering an effective therapeutic amount of the above-mentioned GLP-1 analogue to a patient is provided.

### Advantage of the Invention

The GLP-1 analogue of the present invention has aggregability which is not available in conventional GLP-1 analogues, and by using an association aggregate of the GLP-1 analogue of the present invention, a preventive agent or a therapeutic agent for diabetes, hyperglycemia, a diabetic complication caused by diabetes or hyperglycemia or obesity, showing long-term sustained release, which could not be achieved by conventional GLP-1 analogues can be provided.

### Brief Description of the Drawings

[Fig. 1] Fig. 1 shows the measurement results of CD spectra of WT-GLP-1 before and after stirring.
[Fig. 2] Fig. 2 shows the measurement results of CD spectra of GLP-1 analogue (Peptide 1) of the present invention before and after stirring.
[Fig. 3] Fig. 3 shows the changes in the in vitro release of GLP-1 from a Zinc/GLP-1 complex.
[Fig. 4] Fig. 4 shows the measurement results of CD spectra of GLP-1 analogue (Peptide 3) before and after stirring.
[Fig. 5] Fig. 5 shows scanning electron micrographs of lyophilized powder of aggregate of GLP-1 analogue (Peptide 1) of the present invention obtained by shaking.
[Fig. 6] Fig. 6 shows the changes in the in vitro release of aggregate of GLP-1 analogue (Peptide 1) of the present invention obtained by shaking.
[Fig. 7] Fig. 7 shows the changes in the solubility when zinc chloride was added at various concentrations to each of GLP-1 analogue (Peptide 1) of the present invention and WT-GLP-1.
[Fig. 8-1] Fig. 8-1 shows scanning electron micrographs of lyophilized powder of zinc salt of GLP-1 analogue (Peptide 1) of the present invention.
[Fig. 8-2] Fig. 8-2 shows a scanning electron micrograph of lyophilized powder of zinc salt of GLP-1 analogue (Peptide 1) of the present invention.
[Fig. 9-1] Fig. 9-1 shows scanning electron micrographs of lyophilized powder of zinc salt of WT-GLP-1.
[Fig. 9-2] Fig. 9-2 shows a scanning electron micrograph of lyophilized powder of zinc salt of WT-GLP-1.
[Fig. 10] Fig. 10 shows the changes in the in vitro release of powder of zinc salt of GLP-1 analogue (Peptide 1) of the present invention, and powder of zinc salt of WT-GLP-1.
[Fig. 11] Fig. 11 shows the appearance when a zinc-containing solution of GLP-1 analogue (Peptide 1) of the present invention was added dropwise to a neutral buffer.
[Fig. 12] Fig. 12 shows the changes in the in vitro release of a solidified substance obtained by adding a zinc-containing solution of GLP-1 analogue (Peptide 1) of the present invention dropwise to a neutral buffer.

### Mode for Carrying Out the Invention

Hereinafter, the present invention will be further specifically described.

The present invention relates to an aggregable GLP-1 analogue for sustained-release use, which has a high activity and an unprecedented property of association-aggregability for achieving long-term sustained release.

The synthesis of a peptide or a protein according to the present invention may be carried out by either a conventionally used chemical synthesis method through a solid-phase method or a liquid-phase method, or a method of culturing a recombinant produced by using E. coli or an animal cell as a host. The C-terminus may be in the form of either a carboxylate or an amide, however, from the viewpoint of stability, it is preferably in the form of an amide.

In order to prepare a sustained-release preparation using the present GLP-1 analogue, the GLP-1 analogue is preferably associated and aggregated. For example, the GLP-1 analogue can be associated and aggregated by itself or in the coexistence of a precipitant such as a metal ion. The aggregation method is not particularly limited, however, examples thereof include a method in which the GLP-1 analogue is dissolved (for example, at a concentration of 1 mg/mL) in a buffer with a neutral pH such as PBS, followed by stirring the mixture, a method in which the GLP-1 analogue is dissolved in a solvent under an acidic condition (at a pH of 4 or less) and the pH of the solution is returned to around a neutral pH, followed by stirring the mixture, and a method in which the GLP-1 analogue and a metal ion are dissolved in a solvent under an acidic condition (at a pH of 4 or less) and the pH of the solution is returned to around a neutral pH, followed by stirring the mixture. There is an advantage that in the acidic pH range, the solubility of the GLP-1 analogue is increased therefore, the GLP-1 analogue can be dissolved at a higher concentration thereby to easily make it precipitate.

The term "pharmaceutically acceptable salt" means a salt, which is produced by bringing the GLP-1 analogue into contact with an acid or a base usable in the production of a pharmaceutical preparation and can be used as a pharmaceutical preparation. Here, the base may be a compound (for example, zinc chloride, zinc oxide, zinc acetate, ferric chloride, calcium chloride, calcium oxide, calcium acetate, calcium bromide, calcium carbonate, calcium citrate, calcium hydroxide, calcium lactate, calcium sulfate, aluminum chloride, aluminum hydroxide, aluminum sulfate, potassium chloride, potassium acetate, potassium hydroxide, sodium chloride, sodium acetate, sodium hydrogen phosphate or the like), which provides a metal ion to be used when the GLP-1 analogue is associated and aggregated.

Examples of the metal ion to be used when the GLP-1 analogue is associated and aggregated include zinc, copper, iron, manganese, calcium, nickel, aluminum, sodium and potassium, however, zinc is particularly preferred. As a counter ion, one that promotes the stabilization of the resulting preparation may be selected.

Examples of the method for aggregation or the method for association and aggregation using a precipitant such as a metal ion include again a method generally used for crystallization or amorphization, such as a cooling method, a poor solvent method, an evaporation method, a solvent-mediated phase transition method, a vapor equilibrium method, and the like.

Further, by encapsulating the GLP-1 analogue of the present invention in a drug carrier, a preparation rendering longer sustained release can be obtained. In order to load a drug, any of various known methods can be used. For example, a drug may be formed into a microsphere by an emulsion method together with a biodegradable polymer such as polylactic acid (PLA), polyglycolic acid (PGA) or a copolymer thereof including a copolymer of lactic acid and glycolic acid (PLGA).

Alternatively, an acidic solution containing the GLP-1 analogue in the coexistence of a precipitant such as a metal ion is subcutaneously or topically administered and the GLP-1 analogue can be precipitated and released in a sustained manner at the site of administration such as under the skin. Further, by suitably regulating the concentration of the GLP-1 analogue or the concentration of the metal ion or the like to be added in this acidic solution, the sustained release rate or duration can be controlled.

Further, by conjugating the GLP-1 analogue of the present invention to a water-soluble polymer, the retention thereof in the blood can be improved, and by subcutaneously administering a conjugate per se or by subcutaneously administering a conjugated crosslinked substance, the GLP-1 analogue of the present invention can be released in a sustained manner under the skin or in the blood.

The GLP-1 analogue of the present invention can be administered as a pharmaceutical composition containing one or more pharmaceutically acceptable salts, a diluent, a wetting agent, an emulsifying agent, a dispersant, an adjuvant, a preservative, a buffer, a binder, a stabilizer or the like, by formulating it into an arbitrary suitable form according to the intended administration route. The administration route may be either a parenteral route or an oral route. Examples of the diluent include lactose, sucrose, dextrose, trehalose, sorbitol, mannitol and the like.

The pharmaceutical composition of the present invention can be parenterally administered systemically or topically. For example, intravenous injection such as infusion, intramuscular injection, intraperitoneal injection, subcutaneous injection, intranasal injection or the like can be selected, and the administration method can be appropriately selected according to the age or symptom of the patient. The effective dose thereof varies depending on the administration route or administration frequency. Its blood concentration for obtaining an efficacy while inhibiting the occurrence of an adverse effect (such as nausea or vomiting) is considered to be 2 pM to 1000 pM, preferably 10 pM to 400 pM, more preferably 20 pM to 200 pM, therefore, the dose is preferably adjusted such that the blood concentration thereof falls within the range.

In the case where the GLP-1 analogue or a pharmaceutically acceptable salt thereof of the present invention is administered as a pharmaceutical, the dose thereof as a pharmaceutical for diabetes is preferably adjusted by considering the conditions of the patient such as age or body weight, administration route, nature or severity of the disease or the like. In general, for a human, the amount of active ingredient of the present invention per an adult is in the range of 5 µg to 500 mg per day, however, there is a case where a dose less than the above range is sufficient, and there is also a case where a dose exceeding the above range is necessary. When a large amount thereof is administered, administration is preferably carried out by dividing the daily dose into several portions.

The diabetic complication to which the present invention can be applied is not particularly limited, and examples thereof include diabetic retinopathy, diabetic nephropathy, diabetic neuropathy, diabetic arteriosclerosis, diabetic myocardial infarction, cerebral infarction, diabetic foot lesion and the like.

According to the present invention, a long-lasting pharmaceutical for diabetes, which cannot be obtained by a conventional method, can be provided.

### Examples

Hereinafter, preferred Examples of the present invention will be described in further detail, however, the present invention is not limited to these Examples.

### [Example 1] Biological activity of GLP-1 analogue

A GLP-1 analogue (Peptide 1, SEQ ID NO: 1) according to the present invention and a wild type GLP-1(7-37) (WT-GLP-1, SEQ ID NO: 2), were obtained by a solid-phase synthesis method (Peptide Institute, Inc. and Shimadzu Corporation).

Human GLP-1 receptor-expressing HEK293 cells were inoculated into a 96-well plate at a cell density of 1 x 10⁴ cells/well, and cultured for 3 days. The obtained cultured cells were treated with 0.5 mmol/L IBMX for 30 minutes, and a sample was added thereto to give a final concentration of 1 x 10⁻⁷ to 3.8 x 10⁻¹³ mol/L. Then, after the reaction was allowed to proceed for 30 minutes, the cells were lysed. The concentration of cAMP in the resulting cell lysate was determined using cAMP-Screen System (Applied Biosystems). The sequence of each sample is shown in Table 1, and the EC50 value for cAMP production of each sample is shown in Table 2.

**[[Table 1]**

| | |
|---|---|
| ID No. | Sequence 7--10-----20-----30-----37 |
| WT | HA EGTFTSDVSSYLEGQAAKEF\|A WLVKGRG (SEQ ID NO: 2) |
| Peptide 1 | H\|G\|EGTFTSDVSSYLEGQAAKEFI\|R\|WLVKGRG |

[Table 2]

**Table 2: In vitro biological activity of GLP-1 analogue**

| | |
|---|---|
| ID No. | EC50 (M) |
| WT | 1.23 x 10⁻¹⁰ |
| Peptide 1 | 9.02 x 10⁻¹¹ |

The GLP-1 analogue (Peptide 1) of the present invention was found to maintain an activity substantially equivalent to that of WT-GLP-1.

### [Example 2] Evaluation for aggregability of GLP-1 analogue

Each of the GLP-1 analogue and WT-GLP-1 in Example 1 was dissolved in PBS at a concentration of 1 mg/mL, and the amount of monomer was determined by RP-HPLC. 450 µL of each of the same solutions was placed into a glass vial along with a stirring bar, and the solution was stirred with a stirrer at room temperature for 20 hours. After stirring, the absorbance of the solution at 350 nm was determined (DU640, manufactured by Beckman). As for the supernatant after centrifugation, the monomer quantification by RP-HPLC and CD measurement (J-725, manufactured by Jasco Co.) were carried out.

### RP-HPLC conditions

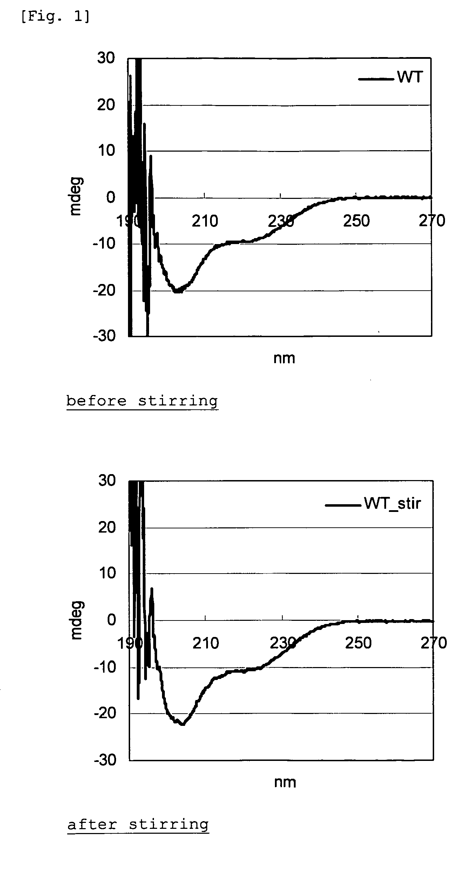

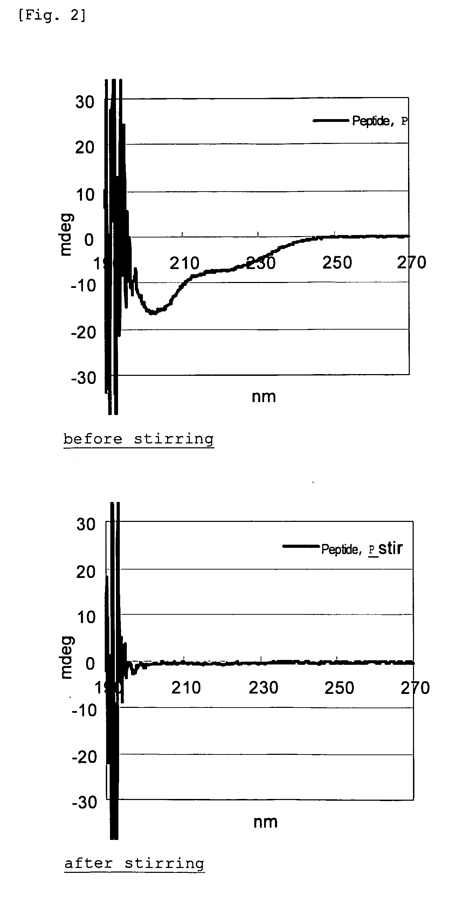

System: NanoSpace SI-2 (manufactured by Shiseido Co., Ltd.)
Column: Capcell PAK C18, 1 mm x 75 mm (manufactured by Shiseido Co., Ltd.)
Flow rate: 0.1 mL/min
Detection: UV (215 nm/280 nm)
Eluent A: water/acetonitrile/TFA = 949/50/1
Eluent B: water/acetonitrile/TFA = 50/949/1
Elution method: Gradient from 80/20 to 20/80 (Eluent A/Eluent B)
The CD spectra are shown in Fig. 1 and Fig. 2, and the results are shown in Table 3.

[Table 3]

**Table 3: Evaluation for aggregability of GLP-1 analogue**

| | UV 350 nm Absorbance | RP-HPLC Amount of residual monomer (%) |
|---|---|---|
| WT | 0.13 | 90 |
| Peptide 1 | 1.42 | 10 |

As for the GLP-1 analogue (Peptide 1) of the present invention, white turbidity was easily confirmed visually, and an increase in the absorbance (turbidity) at 350 nm and a decrease in the ratio of residual monomer due to precipitation were observed. It was confirmed that the aggregability of Peptide 1 was significantly increased compared with that of WT-GLP-1.

### [Example 3] Evaluation for sustained release of aggregate of GLP-1 analogue

The quantification of GLP-1 analogue was carried out using RP-HPLC.
System: Waters Alliance 2690/2487
Column: YMC-ODS A, 2.0 mm x 250 mm (YMC)
Flow rate: 1 mL/min
Detection: UV (215 nm/280 nm)
Eluent A: water/acetonitrile/TFA = 949/50/1
Eluent B: water/acetonitrile/TFA = 49/950/1
Elution method: Gradient from 65/35 to 0/100 (Eluent A/Eluent B)

### [Example 3-1] Preparation of Zinc/GLP-1

400 µg of each of WT-GLP-1 and GLP-1 analogue (Peptide 1) was dissolved in a 1 mM HCl solution (200 µL, pH: 2.8) containing 2 mg/mL zinc chloride. 100 µL of each of the resulting solutions was added to E-tube and diluted with a 1 mM HCl solution, whereby a 1 mg/mL solution was prepared. Then, 20 µL of PB (pH: 8.9, 100 mM) was added thereto, whereby a complex of Zinc/GLP-1 was formed. After the resulting mixture was centrifuged at 15,000 rpm for 5 minutes, the supernatant was removed, whereby a Zinc/GLP-1 complex was obtained.

### [Example 3-2] In vitro release test of GLP-1 from Zinc/GLP-1 complex

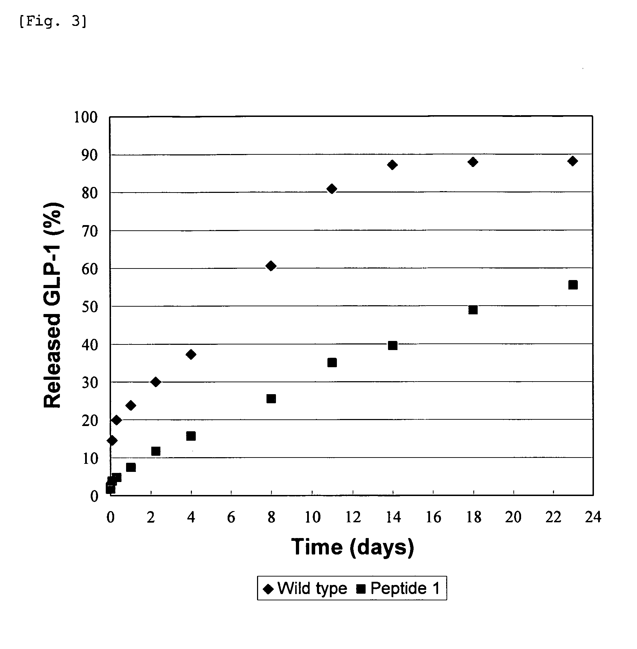

PBS (0.5 mL, pH: 7.4) was added to E-tube containing the Zinc/GLP-1 complex at 37°C. The E-tube was centrifuged at different time points, and the supernatant was sampled, and 0.5 mL of PBS was freshly added thereto. 20 µL of 0.2 M HCL was added to 200 µL of the sample, and quantification was carried out by RP-HPLC. The results are shown in Fig. 3.

As for WT-GLP-1, the release was observed until day 14, however, the release rate of Peptide 1 was lower than that of WT-GLP-1, and the release amount was about 55% on day 23. The Zinc/Peptide 1 complex showed a longer sustained release than the Zinc/WT-GLP-1 complex.

### [Comparative example 1] Evaluation for aggregability of GLP-1 analogue

A GLP-1 analogue (Peptide 3, SEQ ID NO: 3) was obtained by a solid-phase synthesis method (manufactured by Shimadzu Corporation).

**[Table 4]**

| | | |
|---|---|---|
| ID No. | Sequence 7--10-----20-----30-----37 | pl |
| WT | HA EGTFTSDVSSYLEGQAAKEF\|AWLVKGRG | 5.13 |
| Peptide 1 | H\|G\|EGTFTSDVSSYLEGQAAKEFI\|R\|WLVKGRG | 7.80 |
| Peptide 3 | H\|G\|EGTFTSDV\|K\|SYLEGQAAKEF\|AWLVKGRG (SEQ ID NO: 3) | 7.80 |

The aggregability of Peptide 3 was evaluated in the same manner as the method shown in Example 2.

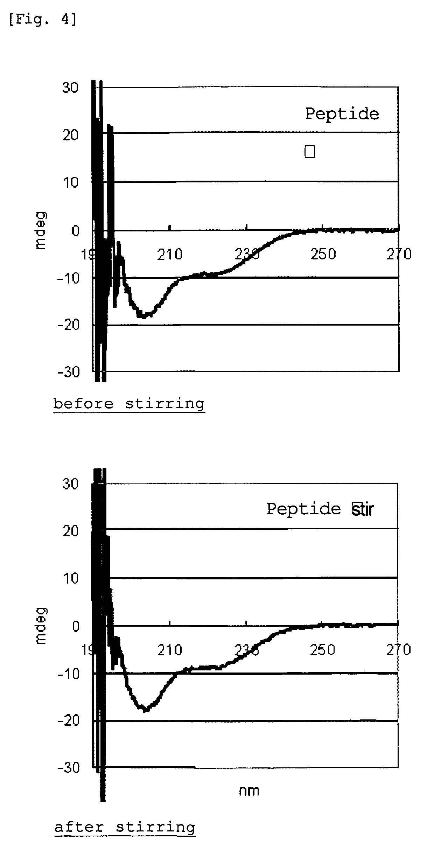

The CD spectra are shown in Fig. 4, and the results are shown in Table 5.

[Table 5]

**Table 5: Evaluation for aggregability of GLP-1 analogue**

| | UV 350 nm Absorbance | RP-HPLC Amount of residual monomer (%) |
|---|---|---|
| WT | 0.13 | 90 |
| Peptide 3 | 0.06 | 95 |

Peptide 3 has an isoelectric point nearly equal to that of Peptide 1, however, an increase in the aggregability compared with that of WT-GLP-1 was not observed.

### [Example 4] Preparation of aggregate of GLP-1 analogue and evaluation for sustained release thereof

A GLP-1 analogue (Peptide 1, SEQ ID NO: 1) according to the present invention was obtained by a solid-phase synthesis method (manufactured by AMERICAN PEPTIDE COMPANY, INC.).

The quantification of GLP-1 analogue was carried out using RP-HPLC (the analytical conditions are shown below).

System: Waters Alliance 2790/2487
Column: Cadenza CD18-C (3 µm) 3.0 mm x 50 mm (manufactured by Imtakt)
Flow rate: 0.75 mL/min
Detection: UV (280 nm)
Eluent A: Milli-Q water containing 0.01% w/v TFA
Eluent B: acetonitrile containing 0.01% w/v TFA
Elution method: Linear gradient from 80/20 to 50/50 (Eluent A/Eluent B)

### [Example 4-1] Preparation of aggregate of GLP-1 analogue

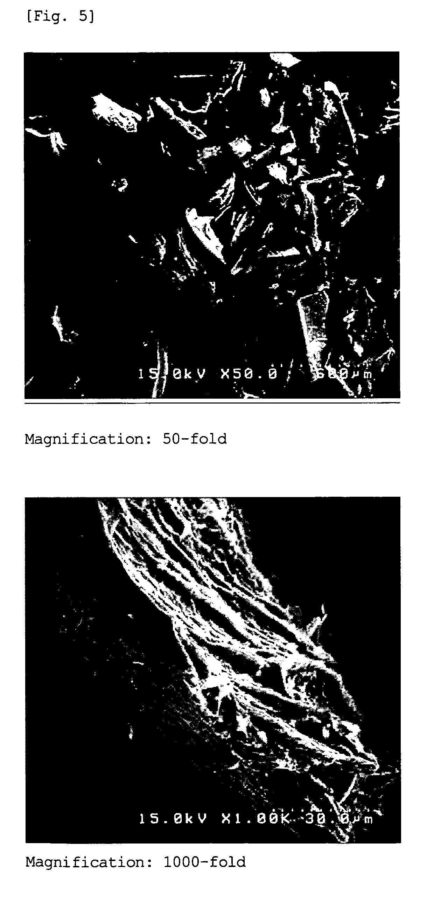

20 mg of Peptide 1 was dissolved in 20 mM phosphate buffer (pH 7.4) at a concentration of 1 mg/mL, and the resulting solution was dispensed in 1 mL aliquots into 20 Eppendorf tubes. The concentration of peptide was determined by RP-HPLC in one of the 20 Eppendorf tubes. The remaining 19 Eppendorf tubes were shaked in a shaker at room temperature for 24 hours. The solution after shaking was apparently turbid in white. After shaking, the solution was subjected to a centrifugation procedure (10,000 g, 3 min) and decantation, and the white precipitate and supernatant were separated. When the concentration of peptide in the supernatant was determined by RP-HPLC, the ratio thereof to the concentration of peptide before shaking was found to be 0.6% (6 µg/mL), which suggested that 99.4% of peptide was aggregated. The recovered white precipitate was washed three times with Milli-Q water, and the respective precipitates were brought together into one. The washing liquid was removed by decantation, and the residue was lyophilized, whereby white powder of an aggregate of GLP-1 analogue was obtained. The weight of the obtained white powder was measured and found to be 14.0 mg. The scanning electron micrographs of the obtained powder are shown in Fig. 5. The aggregate of GLP-1 analogue was in the form of a powder with a size of about 50 to around 60 µm and had a multi-layered structure in the cross section.

### [Example 4-2] In vitro evaluation for elution rate of aggregate of GLP-1 analogue

About 0.5 mg of the white powder of aggregate of GLP-1 analogue obtained in Example 4-1 was weighed out and placed in each of 8 dialysis chambers (EasySep, MWCO: 14,000, manufactured by TOMY SEIKO Co., Ltd.). One chamber was cryopreserved as a standard. To the remaining 7 chambers, 1 mL of PBS (pH 7.4) was added, and the powder was dispersed well with a vortex mixer. Then, a cap of dialysis membrane was attached to each chamber, and the chamber was placed such that the cap faced downward. The chambers were shaked well so as to prevent the dispersed powder from adhering to the upper wall of the container and then installed in a float. Then, equilibrium dialysis was carried out at 37°C against 1 L of PBS (pH 7.4) containing 0.01% w/v sodium azide. After 3 hours, 1 day, 2 days, 1 week, 2 weeks, 3 weeks and 4 weeks, the dialysis chamber was recovered, and the precipitate of aggregate of GLP-1 analogue and supernatant were separated by a centrifugation procedure, and the supernatant was removed by decantation. The recovered precipitate was lyophilized.

To each of the standard and the lyophilized samples, 120 µL of 0.05 N hydrochloric acid containing 0.05% w/v Tween 80, 40 µL of DMSO and 40 µL of acetonitrile were added to dissolve each substance, and the amount of peptide was determined by RP-HPLC.

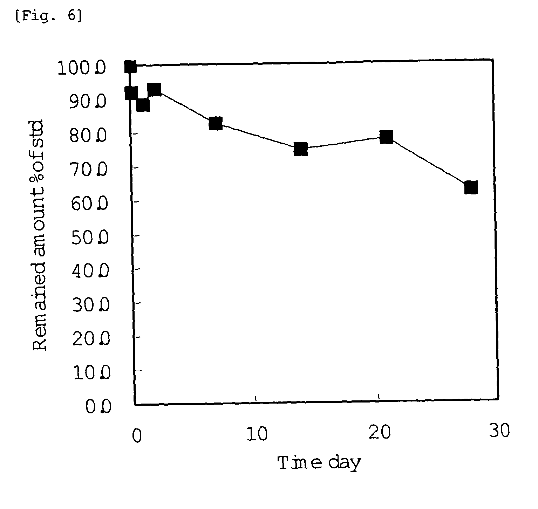

The amount of peptide remaining in the dialysis chamber was calculated as a percentage to that of standard. The results of plotting the calculated amount against time are shown in Fig. 6.

The elution of aggregate of GLP-1 peptide was very slow at 37°C in PBS, and an almost zero-order elution pattern was observed, and the amount of elution for 4 weeks was about around 35%.

### [Example 5] Preparation of powder of zinc salt of GLP-1 analogue and evaluation for sustained release thereof

As the GLP-1 analogue (Peptide 1, SEQ ID NO: 1) according to the present invention, one obtained in Example 4 was used, and as the wild-type GLP-1(7-37) (WT-GLP-1, SEQ ID NO: 2), one obtained in Example 1 was used.

The quantification of GLP-1 analogue was carried out by the method shown in Example 4.

The quantification of the content of zinc in the zinc salt of GLP-1 analogue was carried out by the Zincon method shown below.

### Zincon method

13.58 mg of zinc chloride (MW: 136.3) was dissolved in 24.91 mL of Milli-Q water, whereby a 4 mM zinc chloride standard was prepared. The zinc chloride standard was diluted with Milli-Q water, whereby six 2-fold serial dilutions were prepared. Then, 13.21 mg of Zincon (MW: 462.4) was dissolved in 285.7 µL of 1 N aqueous sodium hydroxide solution, and the resulting solution was diluted to 50-fold with Milli-Q water, whereby a 2 mM Zincon standard was prepared.

To 20 µL of each of the serial dilutions of zinc chloride standard, 960 µL of 100 mM borate buffer (pH 9.0) and 20 µL of the Zincon standard were added and well mixed therein, and the resulting mixture was let stand at room temperature for 30 minutes. The absorbance of these samples at 617 nm was determined with a spectrophotometer, and a curve obtained by plotting the absorbance against the concentration of zinc chloride was used as a calibration curve.

A zinc salt sample with an unknown zinc concentration was dissolved in 10 mM hydrochloric acid such that the resulting concentration falls within the range of the calibration curve. To 20 µL of the solution with an unknown concentration, 960 µL of 100 mM borate buffer (pH 9.0) and 20 µL of the Zincon standard were added and well mixed therein, and the resulting mixture was let stand at room temperature for 30 minutes. The absorbance thereof at 617 nm was determined with a spectrophotometer, and the quantification of the zinc concentration was carried out based on the previously constructed calibration curve.

### [Example 5-1] Study of preparation condition for zinc salt of GLP-1 analogue

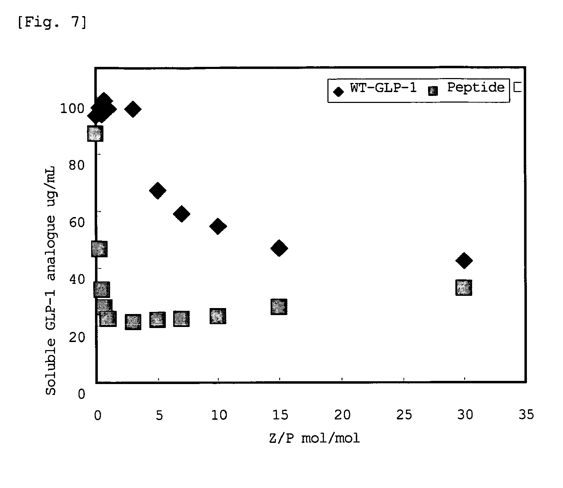

Each of Peptide 1 and WT-GLP-1 was dissolved in Milli-Q water to give a final concentration of 1 mg/mL. Further, zinc chloride was dissolved in Milli-Q water to give a final concentration of 0.1 mg/mL. 1 M HEPES (N-[2-hydroxyethyl]piperazine-N'-[2-ethanesulfonic acid]) buffer (pH 7.4) was separately prepared. To each of the aqueous solutions of peptide, the HEPES buffer, Milli-Q water and the aqueous solution of zinc chloride were sequentially added, whereby samples were prepared such that the final volume was 1 mL, the final concentration of peptide was 100 µg/mL, the final concentration of HEPES (pH 7.4) was 100 mM, and the ratio of zinc chloride to the mole number of peptide became various ratios (Z/P = 0 to 30). These samples were let stand at room temperature (25°C) for 18 hours. Each mixture was centrifuged (10,000 g, 3 min), and the concentration of peptide in the supernatant was determined by RP-HPLC. The results are shown in Fig. 7.

A zinc salt of Peptide 1 was formed by adding zinc chloride in a smaller amount compared with that for WT-GLP-1, and the solubility thereof in the buffer (pH 7.4) was significantly decreased compared with a free form (before adding zinc chloride). The ratio of added zinc to peptide when the solubility thereof was the lowest was Z/P = around 1 in the case of Peptide 1, and Z/P = 15 to 30 in the case of WT-GLP-1. It was suggested that the solubility of the zinc salt of Peptide 1 at this time was lower than that of the zinc salt of WT-GLP-1.

### [Example 5-2] Preparation of zinc salt of Peptide 1 for evaluation of sustained release

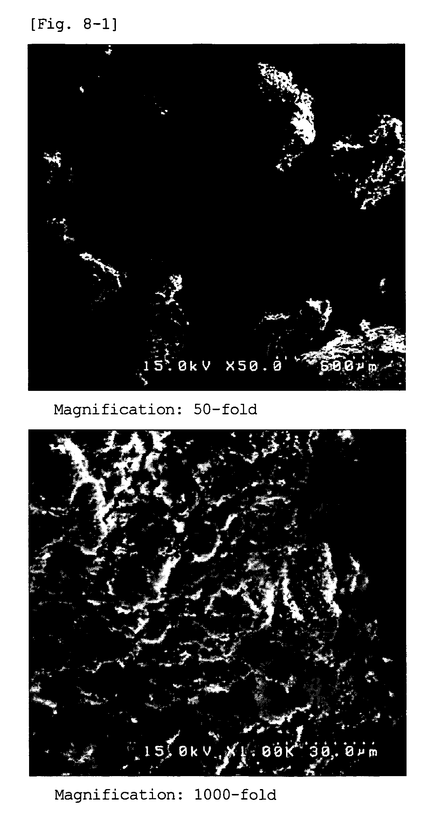

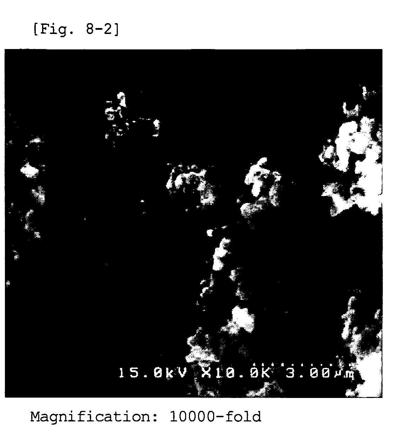

Peptide 1 was dissolved in Milli-Q water to give a final concentration of 5 mg/mL. Further, zinc chloride was dissolved in Milli-Q water to give a final concentration of 1 mg/mL. 1 M HEPES buffer (pH 7.4) was separately prepared. To the aqueous solution of peptide, the HEPES buffer, Milli-Q water and the aqueous solution of zinc chloride were sequentially added, whereby a sample was prepared such that the final volume was 30 mL, the final concentration of peptide was 1 mg/mL, the final concentration of HEPES (pH 7.4) was 100 mM, and the ratio of zinc chloride to Peptide 1 became Z/P = 1. The resulting solution was let stand at room temperature (25°C) for 18 hours. The solution after being let stand was obviously turbid in white. The solution was subjected to a centrifugation procedure (10,000 g, 3 min) and decantation, whereby the white precipitate and supernatant were separated. When the concentration of peptide in the supernatant was determined by RP-HPLC, the ratio thereof to the concentration of peptide in the case where zinc chloride was not added was found to be 1.6%, which suggested that 98.4% of peptide was precipitated as a zinc salt. After the recovered white precipitate was washed three times with Milli-Q water, the washing liquid was removed by decantation, and the residue was lyophilized, whereby white powder of zinc salt of Peptide 1 was obtained. The weight of the obtained white powder was measured and found to be 23.1 mg. The content of zinc in the white powder was 0.8% w/w. The scanning electron micrographs of the obtained powder are shown in Fig. 8. The zinc salt of Peptide 1 was in the form of a powder with a size of about 150 to around 1000 µm, and based on the observation of the surface shape, it was suggested that the zinc salt of Peptide 1 was an aggregate of fine particles with a size of about 200 nm.

### [Comparative example 2] Preparation of zinc salt of WT-GLP-1 for evaluation of sustained release

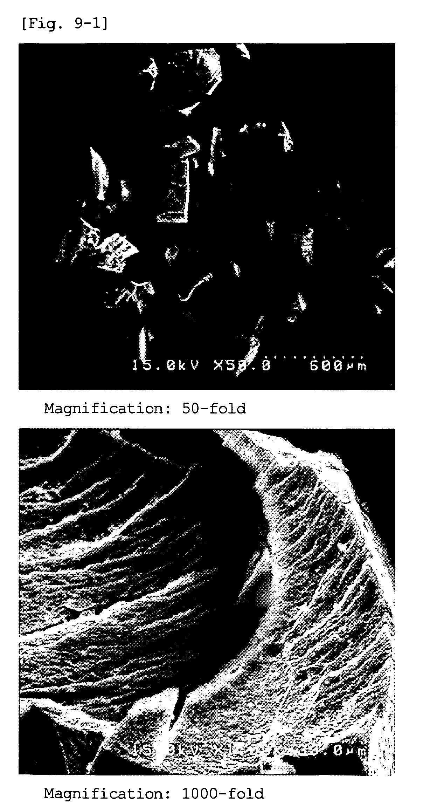

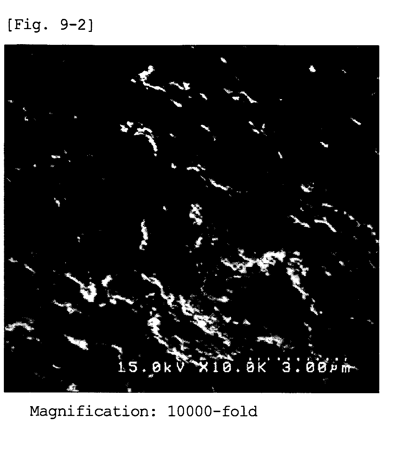

WT-GLP-1 was dissolved in Milli-Q water to give a final concentration of 5 mg/mL. Further, zinc chloride was dissolved in Milli-Q water to give a final concentration of 1 mg/mL. 1 M HEPES buffer (pH 7.4) was separately prepared. To the aqueous solution of peptide, the HEPES buffer, Milli-Q water and the aqueous solution of zinc chloride were sequentially added, whereby a sample was prepared such that the final volume was 30 mL, the final concentration of peptide was 1 mg/mL, the final concentration of HEPES (pH 7.4) was 100 mM, and the ratio of zinc chloride to WT-GLP-1 became Z/P = 15. The resulting solution was let stand at room temperature (25°C) for 18 hours. The solution after being let stand was obviously turbid in white. The solution was subjected to a centrifugation procedure (10,000 g, 3 min) and decantation, whereby the white precipitate and supernatant were separated. When the concentration of peptide in the supernatant was determined by RP-HPLC, the ratio thereof to the concentration of peptide in the case where zinc chloride was not added was 4.3%, which suggested that 95.7% of peptide was precipitated as a zinc salt. After the recovered white precipitate was washed three times with Milli-Q water, the washing liquid was removed by decantation, and the residue was lyophilized, whereby white powder of zinc salt of WT-GLP-1 was obtained. The weight of the obtained white powder was measured and found to be 21.9 mg. The content of zinc in the white solid was 1.8% w/w. The scanning electron micrographs of the obtained powder are shown in Fig. 9. The zinc salt of WT-GLP-1 was in the form of a powder with a size of about 200 to around 600 µm and had a multi-layered structure in the cross section, and it was suggested that the zinc salt of WT-GLP-1 had a solid structure which was apparently different from that of the powder of zinc salt of Peptide 1 in Example 5-2.

### [Example 5-3] In vitro evaluation for elution rate of zinc salt of GLP-1 analogue

About 0.5 mg of each of the white powders of the zinc salts of GLP-1 analogues obtained in Example 5-2 and Comparative example 2 was weighed out and placed in each of 8 dialysis chambers (EasySep, MWCO: 14,000). One chamber was cryopreserved as a standard for each specimen. To the remaining 7 chambers, 1 mL of 10 mM HEPES buffer (pH 7.4) containing 150 mM sodium chloride was added, and the powder was dispersed well with a vortex mixer. Then, a cap of dialysis membrane was attached to each chamber, and the chamber was placed such that the cap faced downward. The chambers were shaked well so as to prevent the dispersed powder from adhering to the upper wall of the container and then installed in a float. Then, equilibrium dialysis was carried out at 37°C against 1 L of 10 mM HEPES (pH 7.4) containing 0.01% w/v sodium azide and 150 mM sodium chloride. After 3 hours, 1 day, 2 days, 1 week, 2 weeks, 3 weeks and 4 weeks, the dialysis chamber was recovered, and the precipitate of zinc salt of GLP-1 analogue and supernatant were separated by a centrifugation procedure, and the supernatant was removed by decantation. The recovered precipitate was lyophilized.

To each of the standards and the lyophilized samples, 120 µL of 0.05 N hydrochloric acid containing 0.05% w/v Tween 80, 40 µL of DMSO and 40 µL of acetonitrile were added to dissolve each substance, and the amount of peptide was determined by RP-HPLC.

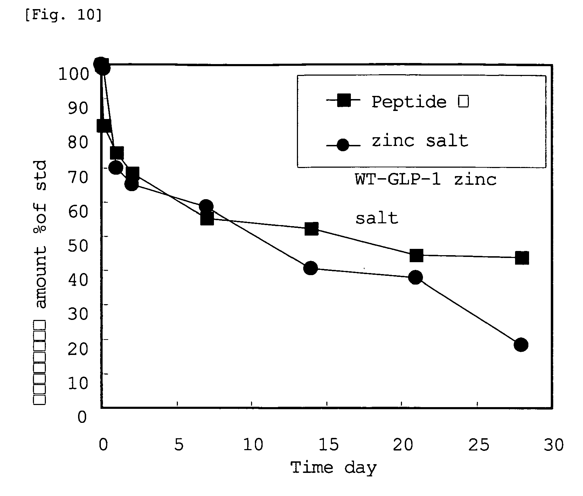

The amount of peptide remaining in the dialysis chamber was calculated as a percentage to that of standard. The results of plotting the calculated amount against time are shown in Fig. 10.

The elution of zinc salt of GLP-1 analogue showed a biphasic elution pattern at 37°C in 10 mM HEPES (pH 7.4). About 30% of zinc salt of GLP-1 analogue was eluted within one day, and an almost zero-order elution pattern was observed for 4 weeks thereafter.

### [Example 6] Solidification behavior due to pH change in zinc-containing solution of GLP-1 analogue and evaluation for sustained release thereof

As the GLP-1 analogue (Peptide 1, SEQ ID NO: 1) according to the present invention, one obtained in Example 4 was used.

The quantification of GLP-1 analogue was carried out by the method shown in Example 4.

### [Example 6-1] Solidification behavior due to pH change in zinc-containing solution of GLP-1 analogue

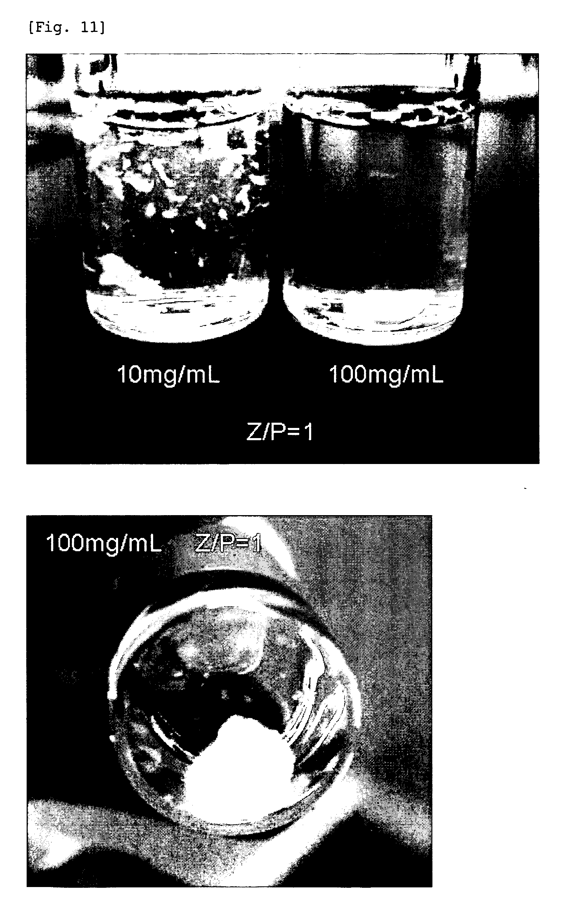

Peptide 1 was dissolved in 10 mM hydrochloric acid to give a final concentration of 150 mg/mL. Further, zinc chloride was dissolved in 10 mM hydrochloric acid to give a final concentration of 15 mg/mL. To the aqueous solution of peptide, 10 mM hydrochloric acid and a hydrochloric acid solution of zinc chloride were sequentially added, whereby samples were prepared such that the final volume was 50 µL, the final concentration of peptide was 10 mg/mL (1% w/v) and 100 mg/mL (10% w/v), the final concentration of hydrochloric acid was 10 mM, and the ratio of zinc chloride to the mole number of peptide became Z/P = 1. 50 µL of each of the resulting zinc-containing solutions of Peptide 1 was slowly added dropwise to 2 mL of 500 mM HEPES (pH 7.4), and the appearance was observed. The results are shown in Fig. 11.

When the zinc-containing hydrochloric acid solution of Peptide 1 became neutral (pH 7.4) with the buffer, a white solid was promptly formed spontaneously. While when the concentration of peptide was 1% w/v, fluffy fine particles were formed, when the concentration of peptide was 10% w/v, one block of solid was formed. This suggested that in the case where a zinc-containing hydrochloric acid solution of Peptide 1 was administered to a neutral environment (for example, under the skin or topical area of a living body or the like), a solid was spontaneously formed accompanying the pH change, and the solidification behavior in which the particle size varies depending on the concentration of peptide occurred.

### [Example 6-2] In vitro evaluation for elution rate of zinc-containing solution of GLP-1 analogue

1 mL of 500 mM HEPES (pH 7.4) containing 150 mM sodium chloride was dispensed into each of 14 dialysis chambers (EasySep, MWCO: 14,000). Then, each of the zinc-containing solutions of 1% w/v Peptide 1 and 10% w/v Peptide 1, both of which were obtained in the same manner as in Example 6-1, was slowly added dropwise to 7 chambers in each case in an amount of 100 µL in the case of the former, and 10 µL in the case of the latter (1 mg in terms of the amount of peptide in each case), and spontaneous solidification behavior was observed. Further, each of the zinc-containing solutions of GLP-1 analogue was dispensed into the dialysis chambers into which the HEPES buffer was not dispensed in an amount of either 100 µL or 10 µL, and cryopreserved as a standard. After the respective 7 samples were lightly dispersed with a vortex mixer, a cap of dialysis membrane was attached to each of the chambers, and then, the chambers were placed such that the cap faced downward. The chambers were shaked so as to prevent the dispersed powder from adhering to the upper wall of the container and then installed in a float. Then, equilibrium dialysis was carried out at 37°C against 2 L of 10 mM HEPES (pH 7.4) containing 0.01% w/v sodium azide and 150 mM sodium chloride. After 3 hours, 1 day, 2 days, 1 week, 2 weeks, 3 weeks and 4 weeks, the dialysis chamber was recovered, and the precipitate of zinc salt of GLP-1 analogue and supernatant were separated by a centrifugation procedure, and the supernatant was removed by decantation. The recovered precipitate was lyophilized.

To each of the standards and the lyophilized samples, 120 µL of 0.05 N HCl containing 0.05% w/v Tween 80, 40 µL of DMSO and 40 µL of acetonitrile were added to dissolve each substance, and the amount of peptide was determined by RP-HPLC.

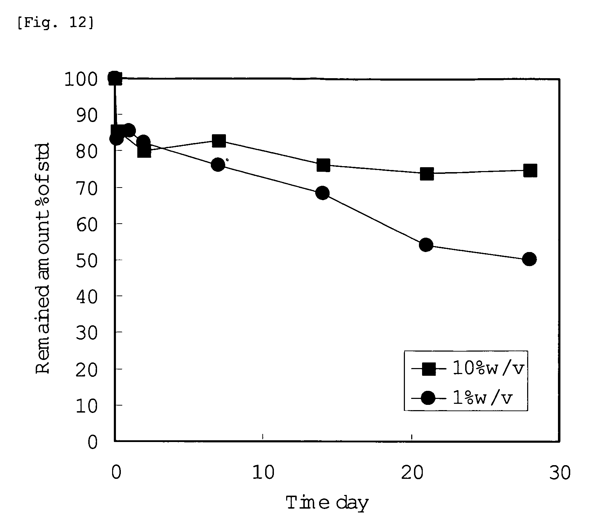

The amount of peptide remaining in the dialysis chamber was calculated as a percentage to that of standard. The results of plotting the calculated amount against time are shown in Fig. 12.

The elution of zinc salt of Peptide 1 spontaneously solidified from the zinc solution showed a biphasic elution pattern at 37°C in 10 mM HEPES (pH 7.4). About 20% of zinc salt of Peptide 1 was eluted within one day, and an almost zero-order elution pattern was observed for 4 weeks thereafter. The amount of elution was about around 50% in the case of 1% w/v solution and about 25% in the case of 10% w/v solution. In combination with the results of Example 6-1, in the zinc-containing solution of Peptide 1, the solidification behavior and elution rate varied depending on the concentration of peptide in the case where solidification was carried out in a neutral environment.

## Claims

1. A GLP-1 analogue wherein alanine at the 30-position (Ala³⁰) of glucagon-like peptide-1 (GLP-1) or a GLP-1 analogue is Lys (Lys³⁰) or arginine (Arg³⁰) or a pharmaceutically acceptable salt thereof.

2. The GLP-1 analogue or a pharmaceutically acceptable salt thereof according to claim 1, wherein the alanine at the 30-position (Ala³⁰) of glucagon-like peptide-1 (GLP-1) or a GLP-1 analogue has been substituted with arginine (Arg³⁰).

3. The GLP-1 analogue or a pharmaceutically acceptable salt thereof according to claim 1, wherein the substituted GLP-1 or GLP-1 analogue is selected from GLP-1(7-37), GLP-1(7-36)NH₂, [Gly⁸]-GLP-1(7-37), [Gly⁸]-GLP-1(7-36)NH₂, [Ser⁸]-GLP-1(7-37), [Ser⁸]-GLP-1(7-36)NH₂, [Val⁸]-GLP-1(7-37), [Val⁸]-GLP-1(7-36)NH₂, [Leu⁸]-GLP-1(7-37), [Leu⁸]-GLP-1(7-36)NH₂, [Ile⁸]-GLP-1(7-37), [Ile⁸]-GLP-1(7-36)NH₂, [Thr⁸]-GLP-1(7-37), [Aib⁸]-GLP-1(7-36)NH₂, [Aib⁸,³⁵]-GLP-1(7-36)NH₂ and [Thr⁸]-GLP-1(7-36)NH₂.

4. The GLP-1 analogue or a pharmaceutically acceptable salt thereof according to claim 1, wherein the amino acid sequence thereof is represented by His-Gly-Glu-Gly-Thr-Phe-Thr-Ser-Asp-Val-Ser-Ser-Tyr-Leu-Glu-Gly-Gln-Ala-Ala-Lys-Glu-Phe-Ile-Arg-Trp-Leu-Val-Lys-Gly-Arg-Gly (SEQ ID NO: 1).

5. The GLP-1 analogue or a pharmaceutically acceptable salt thereof according to any one of claims 1 to 4, which is associated and aggregated.

6. The GLP-1 analogue or a pharmaceutically acceptable salt thereof according to claim 5, which contains one or more metal ions selected from zinc, copper, iron, manganese, calcium, nickel, aluminum, sodium and potassium.

7. A pharmaceutical composition comprising the GLP-1 analogue or a pharmaceutically acceptable salt thereof according to any one of claims 1 to 6.

8. The pharmaceutical composition according to claim 7, wherein the associated and aggregated GLP-1 analogue or pharmaceutically acceptable salt thereof is prepared by adding one or more metal ions selected from zinc, copper, iron, manganese, calcium, nickel, aluminum, sodium and potassium.

9. The pharmaceutical composition containing a compound according to claim 7 or 8, which is used for preventing or treating a disease selected from diabetes, hyperglycemia, diabetic complications caused by diabetes or hyperglycemia and obesity.

10. The pharmaceutical composition according to claim 9, wherein the diabetes is insulin-dependent diabetes mellitus (type 1 diabetes) or noninsulin-dependent diabetes mellitus (type 2 diabetes).

11. A method for preventing or treating a disease selected from diabetes such as insulin-dependent diabetes mellitus (type 1 diabetes) or noninsulin-dependent diabetes mellitus (type 2 diabetes), hyperglycemia, diabetic complications caused by diabetes or hyperglycemia and obesity, comprising administering an effective therapeutic amount of the GLP-1 analogue or a pharmaceutically acceptable salt thereof according to any one of claims 1 to 6 to a patient.
